(19) Europäisches Patentamt
European Patent Office
Office européen des brevets

(11) Veröffentlichungsnummer: **0 300 275**
**A2**

(12) # EUROPÄISCHE PATENTANMELDUNG

(21) Anmeldenummer: **88110817.9**

(51) Int. Cl.⁴: **C07C 143/56**

(22) Anmeldetag: **07.07.88**

(30) Priorität: **18.07.87 DE 3723801**

(43) Veröffentlichungstag der Anmeldung:
**25.01.89 Patentblatt 89/04**

(84) Benannte Vertragsstaaten:
**BE CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Blank, Heinz Ulrich, Dr.**
**Am Geusfelde 35**
**D-5068 Odenthal-Glöbusch(DE)**
Erfinder: **Emde, Herbert, Dr.**
**Hardthofstrasse 13**
**D-5000 Köln 80(DE)**
Erfinder: **Schnegg, Peter, Dr.**
**Heidberger Strasse 44**
**D-5068 Odenthal(DE)**

(54) **Verfahren zur Herstellung von Aminoaryl-sulfonsäuren.**

(57) Aminoaryl-sulfonsäuren können durch Umsetzung von Arylaminen und Schwefelsäure bei erhöhter Temperatur und unter Druck hergestellt werden, wobei man gebildetes und gegebenenfalls als Verdünnungswasser vorhandenes Wasser bis zum Ende der Umsetzung im Reaktionsgemisch beläßt.

EP 0 300 275 A2

## Verfahren zur Herstellung von Aminoaryl-sulfonsäuren

Die vorliegende Erfindung betrifft ein Verfahren zur Herstellung von Aminoaryl-sulfonsäuren durch Umsetzung von Arylaminen und Schwefelsäure bei erhöhter Temperatur und unter Druck.

Aromatische Aminosulfonsäuren kann man nach dem sogenannten "Backverfahren" aus Arylaminen und Schwefelsäure bei erhöhter Temperatur erhalten (Helv. Chim. Acta 15 (1932), 1372). Hierbei wird aus zunächst gebildeten Arylammonium-hydrogensulfaten bei erhöhten Temperatur entweder in Substanz oder in einem inerten Lösungsmittel unter Wasseraustritt die entsprechende Aminoaryl-sulfonsäure gebildet; das Wasser wird möglichst rasch und vollständig aus dem Reaktionsgemisch entfernt (Ind. Eng. Chem. 42 (1950), 1746). Dieses bekannte "Backverfahren" liefert bei langen Reaktionszeiten und teilweise mäßigen Ausbeuten häufig dunkelgefärbte Massen, die als unerwünschte Nebenprodukte Aminoaryl-disulfonsäuren sowie isomere Aminoaryl-sulfonsäuren enthalten.

In EP 63 271 ist ein Verfahren zur Herstellung von Aminoaryl-sulfonsäuren beschrieben, bei dem man von der herkömmlichen Variante des "Backverfahrens" dadurch abweicht, daß man entstehendes Reaktionswasser und evtl. vorhandenes Verdünnungswasser nicht möglichst rasch und vollständig aus dem Reaktionsgemisch entfernt, sodern bis kurz vor Ende der Umsetzungsreaktion stets einen Teil dieses Wassers im Reaktionsgemisch beläßt und erst in der letzten Reaktionsphase zur Vervollständigung der Umsetzung das Reaktionswasser vollständig aus dem Reaktionsansatz herausnimmt. Bei diesem Verfahren wird unter Druck gearbeitet. Es zeigt erhebliche Vorteile gegenüber den weiter oben beschriebenen herkömmlichen "Backverfahren".

Es wurde nun überraschend gefunden, daß man weitere Vorteile erzielt, wenn man sich von dem Gedanken der möglichst vollständigen und raschen Entfernung von Wasser aus dem Reaktionsgemisch vollkommen abwendet. Das erfindungsgemäße Verfahren beinhaltet vielmehr die vollständige Belassung des Wassers im Reaktionsgemisch bis zum Ende der Umsetzung. Maximale Unsetzung wird hierbei im Gleichgewichtszustand erreicht. Es ist überraschend, daß dieser Gleichgewichtszustand einen hohen Umsatz des Arylamins beinhaltet. Aus technischwirtschaftlichen Gründen, insbesondere zur Verkürzung der Reaktionszeit, kann die Umsetzung jedoch auch vor Erreichen des Gleichgewichtszustandes beendet werden.

Die vorliegende Erfindung betrifft demnach ein Verfahren zur Herstellung von Aminoaryl-sulfonsäuren durch Umsetzung von Arylaminen und Schwefelsäure bei erhöhter Temperatur und unter Druck, das dadurch gekennzeichnet ist, daß man das gebildete und gegebenenfalls das als Verdünnungswasser vorhandene Wasser bis zum Ende der Umsetzung im Reaktionsgemisch beläßt.

Es ist demnach das kennzeichnende Merkmal des erfindungsgemäßen Verfahrens, daß in einem geschlossenen Reaktionssystem unter Druck gearbeitet wird und während der Umsetzung kein Wasser aus dem Reaktionsgemisch entfernt wird. Als Wasser sei hierbei das in bekannter Weise aus der Umsetzung von Arylaminen und Schwefelsäure entstehende Reaktionswasser verstanden sowie gegebenenfalls zusätzlich eingebrachtes Verdünnungswasser.

Als Druck für die Durchführung des erfindungsgemäßen Verfahrens sei mindestens der Eigendruck des Reaktionsgemisches verstanden, der sich entsprechend dem Fortschritt der Umsetzung einstellt. In einer dem Fachmann geläufigen Weise kann selbstverständlich diesem Eigendruck ein Fremddruck, beispielsweise durch Aufpressen von Stickstoff, Edelgasen oder anderen bezüglich der Umsetzung inerten Gasen, überlagert werden. Die bevorzugte Fahrweise besteht in der bloßen Anwendung des sich entsprechend dem Fortschritt der Umsetzung einstellenden Eigendrucks.

Der sich einstellende Eigendruck, unter dem mindestens gearbeitet wird, sei beispielhaft wie folgt erläutert: Bei der Umsetzung von Anilin mit Schwefelsäure in o-Dichlorbenzol bei 200 °C wird der Eigendruck des o-Dichlorbenzols mit 2,6 bar festgestellt; führt man nun die Umsetzung unter Hinzufügung der genannten Reaktionspartner durch, stellt sich bei den genannten 200 °C durch das entstehende Reaktionswasser ein Druck ein, der rasch über den Druck von 2,6 bar hinausgeht und der schließlich einen Endwert von 6,7 bar erreicht.

Erfindungsgemäß wird im Temperaturbereich von 140-250 °C, bevorzugt 150-230 °C, besonders bevorzugt 180-220 °C gearbeitet.

Erfindungsgemäß kann ohne oder mit Lösungsmittel gearbeitet werden. Die Arbeitsweise mit Lösungsmittel ist bevorzugt. Für den Fall der Arbeitsweise in Gegenwart eines Lösungsmittels seien beispielsweise gegebenenfalls alkyl- und/oder halogensubstituierte Aromaten als geeignet genannt. Hierunter seien beispielsweise Benzol, ein Naphthalin oder Diphenyl verstanden, die bis zu vier Alkylgruppen und/oder Halogenatome, wie Fluor, Chlor oder Brom, als Substituenten tragen können. Als Alkyl-substituenten seien beispielsweise solche mit 1-4, bevorzugt 1-2, besonders bevorzugt 1 C-Atome genannt, wie Methyl,

Ethyl, Propyl, Isopropyl, Butyl oder Isobutyl. Zwei benachbarte Alkylsubstituenten können gemeinsam auch eine Alkylenkette mit 3-5 C-Atomen bilden, wie Trimethylen, Tetramethylen oder Pentamethylen.

Solche Lösungsmittel können sowohl einzeln als auch im Gemisch angewendet werden, beispielsweise technische Dichlorbenzol-, Dichlortoluol-oder Trichlorbenzolgemische.

Als Lösungsmittel können aber auch aliphatische Kohlenwasserstoffe, wie Petroleum, Kerosin, Isododecan oder Dekalin, sowie deren Gemische eingesetzt werden.

Als Lösungsmittel werden bevorzugt alkyl- und/oder halogensubstituierte Benzole eingesetzt. Besonders bevorzugt werden durch 1-3 Halogenatome substituierte Benzole eingesetzt, die daneben noch eine Methylgruppe tragen können. In ganz besonders bevorzugter Weise werden Dichlor- und/oder Trichlorbenzole und/oder -toluole eingesetzt; beispielhaft seien hierbei 1,2-Dichlorbenzol, 1,2,4-Trichlorbenzol und das technische Gemisch der Dichlortoluole genannt.

Das Lösungsmittel wird in einer Menge von 100-2000 ml, bevorzugt 100-500 ml, pro Mol Arylamin eingesetzt. Das molare Verhältnis von Schwefelsäure zu Arylamin beträgt 0,5-1,5:1, bevorzugt 0,7-1,1:1, besonders bevorzugt 0,8-1.05:1. Beispielsweise werden Schwefelsäure und Arylamin im Molverhältnis von etwa 1:1 umgesetzt; auch die entsprechenden Arylammonium-hydrogensulfate in fester Form, als Suspension oder als Schmelze können für das erfindungsgemäße Verfahren eingesetzt werden.

Die Schwefelsäure kann als konzentrierte Schwefelsäure (sogenanntes Monohydrat) oder als verdünnte Schwefelsäure eingesetzt werden. In bevorzugter Weise wird Schwefelsäure mit 96-100 Gew.-% $H_2SO_4$ und ganz besonders bevorzugt das genannte Monohydrat eingesetzt.

Im erfindungsgemäßen Verfahren können Arylamine der allgemeinen Formel

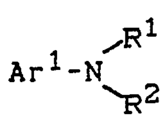 (I)

eingesetzt werden, in der
$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und
$Ar^1$ das gegebenenfalls substituierte Benzol-Naphthalin-, Anthracen-, Naphthochinon- oder Anthrachinongerüst oder das Gerüst eines aromatischen Heterocyclus darstellt.

Als Alkyl sei beilspielsweise ein solches mit 1-8, bevorzugt 1-4, besonders bevorzugt 1-2 C-Atomen genannt, wie Methyl, Ethyl, Propyl, Isopropyl, Butyl, Isobutyl, Hexyl oder Octyl.

Als Aralkyl sei beispielsweise Benzyl, 1-Phenyl-ethyl, 2-Phenyl-ethyl, Naphthyl-methyl, Naphthyl-ethyl, Anthryl-methyl oder Anthryl-ethyl, bevorzugt Benzyl, genannt.

Als Aryl sei beispielsweise Phenyl, substituiertes Phenyl, Naphthyl oder Diphenyl, bevorzugt Phenyl, genannt.

Für den Fall, daß $R^1$ und $R^2$ gemeinsam mit dem N-Atom, das sie substituieren, einen Stickstoffheterocyclus bilden, sei ein solcher mit 4-8, bevorzugt 5-6 Ringgliedern genannt, wie Pyrrolidin oder Piperidin.

Das substituierte Benzol-, Naphthalin-, Anthracen-, Naphthochinon- oder Anthrachinongerüst kann neben der Aminogruppe $-NR^1R^2$ beispielsweise bis zu 3 weitere Substituenten, bevorzugt bis zu 2 Substituenten aufweisen, wobei die Substituenten so angeordnet sein sollen, daß mindestens eine ortho- oder para-Stellung unsubstituiert ist. Als Substituenten seien beispielsweise genannt: Alkyl im Rahmen des genannten Bedeutungsumfanges, Phenyl, Aminophenyl; Halogen, wie Fluor, Chlor oder Brom; weiterhin Hydroxy, $C_1$-$C_4$-Alkoxy, gegebenenfalls substituiertes Amino, $SO_3H$ oder Carboxyl, sowie Alkylsulfonyl oder Arylsulfonyl, wie Methyl-, Ethyl-oder Phenylsulfonyl oder des gegebenenfalls substituierte Benzthiazolylrest. Als Substituenten seien bevorzugt Methyl, Ethyl, Phenyl, Halogen, Hydroxy, Methoxy, Ethoxy oder Amino genannt. Ganz besonders bevorzugt als Substituenten seien Methyl, Chlor, Brom oder Fluor genannt.

Bevorzugte, erfindungsgemäß einsetzbare Arylamine sind solche der Formel

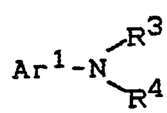 (II),

in der
$Ar^1$ die genannte Bedeutung hat und
$R^3$ und $R^4$ unabhängig voneinander für Wasserstoff oder Alkyl stehen.
Besonders bevorzugte Arylamine für das erfindungsgemäße Verfahren sind solche der Formel

$Ar^1$-$NH_2$ (III),

in der
$Ar^1$ die genannte Bedeutung hat.

Weitere bevorzugte Arylamine für das erfindungsgemäße Verfahren sind solche der Formel

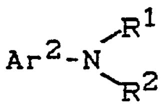 (IV),

in der
$R^1$ und $R^2$ die obengenannte Bedeutung haben und
$Ar^2$ das Benzol- oder Naphthalingerüst darstellt.

Weitere besonders bevorzugte Arylamine für das erfindungsgemäße Verfahren sind solche der Formel

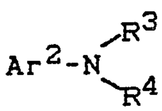 (V),

in der
$R^3$, $R^4$ und $Ar^2$ die genannten Bedeutungen besitzen.

In ganz besonders bevorzugter Weise werden Arylamine der Formel

$Ar^2$-$NH_2$ (VI)

eingesetzt, in der
$Ar^2$ die genannte Bedeutung besitzt und insbesondere das Benzolgerüst darstellt.

Beispiele für im erfindungsgemäßen Verfahren einsetzbare Arylamine sind: Anilin, o-Toluidin, m-Toluidin, p-Toluidin, 2,4-Dimethyl-anilin, 2,3-Dimethyl-anilin, 2,6-Dimethyl-anilin, 2,5-Dimethyl-anilin, N-Methyl-anilin, N-Ethyl-anilin, N,N-Dimethyl-anilin, Diphenylamin, p-Chloranilin, 2,4-Dichlor-anilin, o-Chloranilin, 2,3-Di-chloranilin, 3,5-Dichloranilin, 2,5-Dichloranilin, 2,6-Dichloranilin, m-Chloranilin, 2-Amino-6-chlor-toluol, 2-Amino-5-chlor-toluol, 2-Amino-4-chlor-toluol, 2-Me-thoxyanilin, 4-Methoxyanilin, 2-Ethoxyanilin, 4-Ethoxy-anilin, α-Naphthylamin, p-Phenylendiamin, m-Phenylen-diamin, Amino-diphenyl, 1-Amino-2-hydroxy-naphthalin, 1-Amino-8-hydroxy-naphthalin, 1-Amino-5-hydroxy-naph-thalin, 1,8-Diamino-naphthalin, 1,5-Diamino-naphthalin, 2-Amino-3-hydroxy-naphthalin, 2-Amino-pyridin, 3-Chlor-4-methoxy-anilin, 2-Amino-benzoesäure, 3,4-Dichlor-anilin, o-Fluoranilin, m-Fluoranilin, p-Fluoranilin, 2-Amino-3-chlor-toluol, 3-Amino-2-chlor-toluol, 5-Amino-2-chlor-toluol, 3-Amino-5-chlor-toluol, 3-Amino-4-chlor-toluol, 4-Amino-3-chlor-toluol, 4-Amino-2-chlor-toluol, 3-Amino-6-chlor-benzoesäure, Aminoanthrachinone, wie beilspielsweise 1-Aminoanthrachinon oder 1,5-Diamino-anthrachinon, Benzidin und Dehydrothiotoluidin-sulfonsäure (=2-(4′-Amino-phenyl)-6-methylbenzthiazol-7-sulfonsäure).

Das erfindungsgemäße Verfahren kann diskontinuierlich oder kontinuierlich in Druckreaktoren, wie Autoklaven, Druckkesseln, einer Kaskade von Druckkesseln oder anderen geeigneten Druckreaktoren, wie Schlaufen- oder Röhrenreaktoren durchgeführt werden.

Das erfindungsgemäße Verfahren kann beispielsweise so durchgeführt werden, daß das Arylamin und die Schwefelsäure sowie gegebenenfalls mitverwendetes Lösungsmittel in beliebiger Reihenfolge in einen Druckreaktor gegeben werden. Es ist auch möglich, einige oder alle Reaktionspartner vorher zu vermischen; so kann beispielsweise das Arylamin-hydrogensulfat vorher hergestellt und sodann als solches in den Druckreaktor gegeben werden. Ebenso ist es möglich, das Arylamin und das mitverwendete Lösungsmittel vorher zu vermischen; diese Verfahrensweise wird insbesondere angewendet, wenn das Arylamin bei der Temperatur, bei der es in das Druckgefäß gegeben wird, fest ist. Eine simultane Zugabe von Schwefelsäure und Arylamin in oben beschriebenem molarem Verhältnis wird insbesondere bei der kontinuierlichen Durchführung gewählt. Zur Vermischung der Komponenten werden beilspielsweise Statikmischer oder Ein- oder Mehrstoffdüsen mit innenliegenden oder außenliegenden Vermischungszonen benutzt.

In einer weiteren Variante kann das Lösungsmittel auf die Reaktionstemperatur vorgeheizt werden. Heirzu können sodann das Arylamin und die Schwefelsäure gemeinsam in Form einer Schmelze, Suspension oder Lösung oder einzeln simultan oder nacheinander zugegeben werden.

Das erhaltene Reaktionsgemisch kann, in verschiedener Weise aufgearbeitet werden. Für den Fall, daß ein Lö sungsmittel verwendet wurde, ist es beilspielsweise möglich, die im Lösungsmittel nicht oder nur wenig lösliche Aminoaryl-sulfonsäure durch Abfiltrieren, Zentrifugieren oder ähnliche Techniken vom Lösungsmittel zu trennen. Häufig werden hierbei gut ausgeprägte Kristalle der Aminoaryl-sulfonsäuren erhalten. In einer Variante hiervon können die gewonnenen Kristalle der Aminoaryl-sulfonsäure auch mit Wasser versetzt werden und unter Zugabe einer geeigneten Base in die besser wasserlösliche Salzform übergeführt werden. Als geeignete Base kommen beispielsweise in Frage: Natriumhydroxid, Kaliumhydroxid, Calciumhydroxid, Magnesiumhydroxid, Natriumcarbonat. Kaliumcarbonat, Calciumcarbonat, Magnesiumcarbonat, Ammoniak oder aliphatische Amine. Vorzugsweise werden Natrium- oder Kaliumhydroxid eingesetzt. Diese wäßrige Lösung eines Salzes einer Aminoaryl-sulfonsäure wird sodann zweckmäßigerweise durch Andestillieren von Spuren des verwendeten Lösungsmittels befreit. Die so erhaltene klare, nahezu farblose Salzlösung der Aminoaryl-sulfonsäure kann im allgemeinen ohne jede weitere Reinigung eingesetzt werden. Gegebenenfalls kann diese wäßrige Lösung jedoch auch mit geeigneten Adsorbentien, wie Aktivkohle oder polymeren organischen Adsorptionsmitteln, behandelt werden. Die wäßrige Lösung kann jedoch auch durch Eindampfen auf das in ihr gelöste Salz der Aminoaryl-sulfonsäure aufgearbeitet werden. Die wäßrige Lösung eines Salzes einer Aminoaryl-sulfonsäure kann jedoch auch durch Ansäuern mit einer Mineralsäure, wie Salzsäure oder Schwefelsäure, behandelt werden, wobei die freie, sehr reine Aminoaryl-sulfonsäure ausfällt und, wie oben geschildert, beispielsweise durch Filtration gewonnen werden kann.

Eine wichtige Aufarbeitungsvariante besteht darin, daß das Reaktionsgemisch in der anfallenden Form in Wasser mit einer der obengenannten Basen gegeben wird. Für den Fall einer kontinuierlichen Arbeitsweise kann das kontinuierlich anfallende Reaktionsgemisch simultan mit Wasser und Base oder einer wäßrigen Lösung einer solchen Base in einen geeigneten Behälter, beispielsweise ein Rührgefäß, gegeben werden. Hierbei erhält man zwei flüssige Phasen, von denen die organische das nicht umgesetzte Arylamin sowie gegebenenfalls mitverwendetes organisches Lösungsmittel enthält, während die wäßrige Phase in der oben beschriebenen Weise auf reine Salzlösung, das Salz selbst oder die freie Aminoaryl-sulfonsäure aufgearbeitet wird. Die organische Phase mit dem nicht umgesetzten Arylamin kann, vorzugsweise ohne weitere Reinigung, recyclisiert werden. Diese zuletzt geschilderte Aufarbeitungsvariante ist bevorzugt.

Ein vorteil des Verfahrens liegt in der besonders einfachen technischen Durchführung, da während der Umsetzung kein Wasser aus dem Reaktionsgemisch entnommen wird, was unter anderem eine kontinuierliche Durchführung des Verfahrens wesentlich erleichtert. Durch die Anwendung von Druck im erfindungsgemäßen Verfahren ist die Auswahl an in Frage kommenden Lösungsmitteln sehr groß. Durch die geschilderte Art der Aufarbeitung kann schließlich das gesamte Arylamin recyclisiert und damit letztendlich vollständig umgesetzt werden.

Mit Hilfe des erfindungsgemäßen Verfahrens können sehr reine Aminoaryl-sulfonsäuren hergerstellt werden. Insbesondere ist deren Gehalt an unerwünschten Aminoaryldisulfonsäuren äußerst gering und in jedem Falle kleiner als 2 Gew.-%, vielfach kleiner als 0,5 Gew.-%. Die erfindungsgemäß erhältichen Aminoaryl-sulfonsäuren stellen weiterhin ein sehr helles Produkt dar. Diese Tatsache ist beilspielsweise für den Einsatz von Aminoaryl-sulfonsäuren zur Herstellung von optischen Aufhellern von großer Bedeutung.

Neben der Weiterverarbeitung zu optischen Aufhellern können Aminoaryl-sulfonsäuren als wertvolle Zwischenprodukte für die Herstellung von Pharmazeutika, Schaumstoffen, Netzmitteln, synthetischen Beizmitteln, Gerbstoffen, Reservierungsmitteln, Insektiziden, Appreturmitteln, Weichmachern und polymeren Verdickungsmitteln eingesetzt werden (Ullmanns Encyclopädie der technischen Chemie, 3. Auflage, Band 16 (1965), Seite 561).

Beispiel 1

In einem 4-1-Emaille-Rührautoklaven wurden 651,7 g (7,0 Mol) Anilin in 1400 ml 0-Dichlorbenzol vorgelegt und unter Rühren mit 686,7 g (7,0 Mol) 100 %iger Schwefelsäure versetzt. Darauf wurde der Autoklav geschlossen und das Reaktionsgemisch unter Rühren auf 200 °C erhitzt. Nach 2 1/2 Stunden Reaktionszeit wurde das Reaktionsgemisch abgekühlt, unter Rühren in 1000 ml Wasser gegeben und mit Natronlauge neutralisiert. Man erhielt zwei flüssige Phasen. Die organische Phase wurde nach Ergänzung des Anilins auf den obengenannten Wert einem weiteren Ansatz zugeführt. Aus der wäßrigen Phase wurde durch Ansäuern mit Schwefelsäure reine p-Sulfanilsäure gewonnen. Der Umsatz betrug 64,2 %.

Beispiel 2

Es wurde wie in Beispiel 1 mit den gleichen Einsatzmengen, jedoch bei einer Temperatur von 190 °C gearbeitet. Nach 9 Stunden Reaktionszeit was ein konstanter Absolutdruck von 5,4 bar erreicht, der die Erreichung des Gleichgewichtszustandes anzeigte. Nach dem Abkühlen wurde ein Wohlkristallisierter Rückstand abfiltriert und getrocknet (401,4 g). Der hellgraue Trocknungsrückstand enthielt 71,1 % p-Sulfanilsäure, 27,8 % Anilin-hydrogen-sulfat, 0,5 % o-Sulfanilsäure und 0,1 % Anilin-2,4-disulfonsäure.

Beispiel 3

Es wurde wie in Beispiel 1 mit den gleichen Einsatzmengen und ebenfalls bei einer Temperatur von 200 °C gearbeitet. Nach 4 1/2 Stunden Reaktionszeit wurde ein konstanter Absolutdruck von 6,7 bar erreicht (Gleichgewichtszustand). Nach dem Abkühlen wurde der Feststoff abfiltriert und getrocknet (400,8 g). Der hellgraue Trocknungsrükstand enthielt 71,7 % p-Sulfanilsäure, 25,4 % Anilinhydrogensulfat, 0,5 % o-Sulfanilsäure und 0,06 % Anilin-2,4-disulfonsäure.

Beispiel 4

2-Methylanilin-4-sulfonsäure

In einem 1,3 1-Emaille-Autoklaven wurden 246,4 g 2-Methylanilin in 460 ml o-Dichlorbenzol bei Raumtemperatur vorgelegt und mit 225,5 g 100 %iger Schwefelsäure (Monohydrat) versetzt. Im geschlossenen Autoklaven wurde unter Rühren bei 200 °C Druckkonstanz nach knapp 2 Stunden erreicht.

Nach dem Abkühlen wurden 428 g Feststoff abgesaugt und getrocknet. Die chromatografische Analyse zeigte, bezogen auf den Einsatz an 2-Methylanilin, eine Ausbeute von 89,5 % 2-Methylanilin-4-sulfonsäure. 2-Methylanilin-4,6-disulfonsäure war zu 0,2 % gebildet worden.

Beispiel 5

2-Chloranilin-4-sulfonsäure

Ausgehend von 293,5 g 2-Chloranilin, 460 ml o-Dichlorbenzol und 225,5 g Monohydrat wurde verfahren wie im Beispiel 4. Nach 4,3 Stunden bei 200 °C wurde abgekühlt, abgesaugt und getrocknet. Der isolierte Feststoff wog 489,3 g und enthielt, bezogen auf den Einsatz an 2-Chloranilin, 51,1 % 2-Chloranilin-4-sulfonsäure bei einer Selektivität von 100 %.

Beispiel 6

Naphthionsäure

329,3 g α-Naphthylamin wurden im 1,3 1-Emailleautoklaven in 460 ml o-Dichlorbenzol gelöst und vorsichtig unter Rühren mit 225,5 g Monohydrat versetzt. Nach dem Verschließen des Autoklaven wurde bis zur Druckkonstanz 2 Stunden lang auf 190 °C erhitzt. Die nach dem Erkalten erhaltene Suspension wurde filtriert, der Feststoff getocknet.

Das Produkt (456,0 g) enthielt, bezogen auf den Einsatz an α-Naphthylamin, 34,0 % Naphthionsäure. Die Selektivität der Sulfonierung betrug 91,6 %.

Beispiel 7

p-Sulfanilsäure

In einem verschlossenen Autoklaven wurden 439,7 g gepulvertes Anilinium-hydrogensulfat 7 Stunden lang auf 200 °C erhitzt. Nach dem Abkühlen wurde der Autoklav geöffnet und der Feststoff in 1 n NaOH gelöst (pH 7,2), abgeschiedenes Anilin abgetrennt, restliches Anilin durch Extraktion mit Methylenchlorid (2 x 100 ml) entfernt und die wäßrige Phase zur Trockene eingedampft. Die Analyse des pulvrigen Produktes zeigte eine Ausbeute an p-Sulfanilsäure-Na-salz, bezogen auf eingesetztes Anilin, von 68,5 %. Anilin-2-sulfonsäure war zu 0,7 %, Anilin-2,4-disulfonsäure zu 0,02 % gebildet worden.

Beispiel 8

p-Sulfanilsäure

257,0 g (2,76 mol) Anilin in 460 ml Orthoöl wurden mit 225,5 g (2,3 mol) 100 %iger Schwefelsäure versetzt. Die erhaltene Suspension wurde im 1,3 1-Emaille-Autoklaven 2,5 Stunden auf 200 °C erhitzt. Nach dem Abkühlen wurde abgesaugt und der Filterrückstand getrocknet. Die 475,6 g Feststoff enthielten, bezogen auf die im Unterschuß eingesetzte Schwefelsäure, 80,7 % p-Sulfanilsäure neben unveränderdem Anilinium-hydrogensulfat. Anilin2,4-disulfonsäure war nicht entshanden.

**Ansprüche**

1. Verfahren zur Herstellung von Aminoarylsulfonsäuren durch Umsetzung von Arylaminen und Schwefelsäure bei erhöhter Temperatur und unter Druck ,dadurch gekennzeichnet, daß man das gebildete und das gegebenenfalls als Verdünnungswasser vorhandene Wasser bis zum Ende der Umsetzung im Reaktionsgemisch beläßt.

2. Verfahren nach Anspruch 1, dadurch gekennzeichnet, daß man die Umsetzung bei einer Temperatur von 140 bis 250 °C, bevorzugt 150 bis 230 °C, besonders bevorzugt 180 bis 220 °C durchführt.

3. Verfahren nach Anspruch 1, dadurch gekennzeichnet, daß in Gegenwart eines Lösungs- oder Verdünnungsmittels gearbeitet wird.

4. Verfahren nach Anspruch 3, dadurch gekennzeichnet, daß man 100-2000 ml, bevorzugt 100-500 ml Lösungs-oder Verdünnungsmittel pro Mol Arylamin einsetzt.

5. Verfahren nach Anspruch 1, dadurch gekennzeichnet, daß man Arylamine der Formel

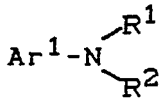

eingesetzt, in der
$R^1$ und $R^2$ unabhängig voneinander Wasserstoff, Alkyl, Aralkyl oder Aryl bedeuten oder beide gemeinsam mit dem N-Atom, das sie substituieren, einen Stickstoffheterocyclus bilden und
$Ar^1$ das gegebenenfalls substituierte Benzol-, Naphthalin-, Anthracen-, Naphthochinon-oder Anthrachinongerüst oder das Gerüst eines aromatischen Heterocyclus darstellt.

6. Verfahren nach Anspruch 6, dadurch gekennzeichnet, daß man Arylamine der Formel

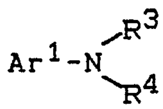

einsetzt, in der
$R^3$ und $R^4$ unabhängig voneinander für Wasserstoff oder Alkyl stehen und
$Ar^1$ das gegebenenfalls substituierte Benzol-, Naphthalin-, Anthracen-, Naphthochinon-oder Anthrachinongerüst oder das Gerüst eines aromatischen Heterocyclus darstellt.

7. Verfahren nach Anspruch 6, dadurch gekennzeichnet, daß man Arylamine der Formel

$Ar^1-NH_2$

einsetzt, in der
$Ar^1$ das gegebenenfalls substituierte Benzol-, Naphthalin-, Anthracen-, Naphthochinon- oder Anthrachinongerüst oder das Gerüst eines aromatischen Heterocyclus darstellt.

8. Verfahren nach Anspruch 1, dadurch gekennzeichnet, daß 0,5-1,5, bevorzugt 0,7-1,1, besonders bevorzugt 0,8-1,05 Mol Schwefelsäure pro Mol Arylamin eingesetzt werden,

9. Verfahren nach Anspruch 1, dadurch gekennzeichnet, daß man das Reaktionsgemisch in Wasser mit einer zur Neutralisation ausreichenden Menge einer Base gibt, die entstehende organische Phase im Verfahren recyclisiert und die wäßrige Phase zur Weiterverarbeitung der Arylamin-sulfonsäure benutzt oder auf reine Arylamin-sulfonsäure oder deren Salz aufarbeitet.